# EUROPEAN PATENT SPECIFICATION

(11) **EP 1 907 089 B1**
(45) Date of publication and mention of the grant of the patent: **23.11.2011**
(21) Application number: 06718718.7
(22) Date of filing: 19.01.2006
(51) Int. Cl.: B01D 15/08, B01L 3/00

(54) **SEPARATION OF PROTEINS BASED ON ISOELECTRIC POINT USING SOLID-PHASE BUFFERS**
TRENNUNG VON PROTEINEN AUF DER BASIS DES ISOELEKTRISCHEN PUNKTS UNTER VERWENDUNG VON FESTPHASENPUFFERN
SEPARATION DE PROTEINES EN FONCTION DE LEUR POINT ISOELECTRIQUE A L'AIDE DE TAMPONS EN PHASE SOLIDE

(30) Priority: 28.07.2005 US 702989 P
(43) Date of publication of application: 09.04.2008
(73) Proprietor: Bio-Rad Laboratories, Inc., Hercules, CA 94547 (US)
(72) Inventor: BOSCHETTI, Egisto, F-78290 Croissy Sur Seine (FR); RIGHETTI, Pier Giorgio, I-20129 Milano (IT); GIROT, Pierre, F-75010 Paris (FR); FORTIS, Frederic, F-95800 Cergy (FR)
(74) Representative: von Kreisler Selting Werner
(86) International application number: PCT/US2006/001686
(87) International publication number: WO 2007/018589

(56) References cited:
- WO-A1-98/22810
- WO-A2-2006/127056
- GB-A- 1 401 585
- US-A- 4 666 856
- US-A- 4 971 670
- US-A1- 2007 039 381
- CRETICH M ET AL: "SEPARATION OF PROTEINS IN A MULTICOMPARTMENT ELECTROLYZER WITH CHAMBERS DEFINED BY A BED OF GEL BEADS" ELECTROPHORESIS, WILEY INTERSCIENCE, DE, vol. 24, no. 4, 1 February 2003 (2003-02-01), pages 577-581, XP009059262 ISSN: 0173-0835
- WENGER P ET AL: "Amphoteric, isoelectric immobiline membranes for preparative isoelectric focusing" JOURNAL OF BIOCHEMICAL AND BIOPHYSICAL METHODS, AMSTERDAM, NL, vol. 14, no. 1, 1 January 1987 (1987-01-01), pages 29-43, XP023454176 ISSN: 0165-022X [retrieved on 1987-01-01]

## Description

Proteomics, the analysis of a set of proteins expressed in a given biological milieu such as cells, tissues, or fluids, has become a major focus of biomedical research. Although the genomes of over one hundred fifty species have been cataloged, a truly functional understanding of the difference between diseased and normal operating states of biological systems requires a correlation of a gene an expressed protein. Gene transcription analysis is incapable of elucidating this correlation, since many genes are regulated indirectly, for example, through processing by the so-called spliceosome, regulation by so-called RNA interference ("RNAi") or by post-translational modification. Thus, to illuminate the gene-product relationship, a catalog of proteins must first be derived.

Before a protein can be cataloged, it must be isolated from its natural environment. Proteins in a mixture can be separated effectively based on their isoelectric points. A molecule's isoelectric point (pI) is the pH at which the molecule carries no net electrical charge. Isoelectric focusing exploits this trait to separate proteins based on their relative content of acidic and basic residues. Briefly, proteins are introduced into a gel composed of polyacrylamide, starch, agarose, etc. which has an established pH gradient or is capable of establishing such a gradient after applying an electrical current. This gradient is established by subjecting a mixture of polyampholytes, small polymers that have different pI values, to electrophoresis. To eliminate a sieving effect, the pores of the gel are made very large. When proteins are introduced to the gel and an electric field is applied, they migrate until they reach a place in the gel where the pH is equal to the isoelectric point of the protein. While isoelectric focusing can resolve proteins that differ in pI value by as little as 0.01, the technique is labor-intensive and unamenable to high through-put processing.

Multicompartment electrolyzers (MEs) with isoelectric membranes were introduced in 1989 for purifying proteins in an electric field. MEs utilize membranes comprising cross-linked copolymers of acrylamide and acrylamido monomers bearing protolytic groups. The use of continuous membranes, however, presents several disadvantages. Due to the mechanical characteristics of polyacrylamide, the gel must adhere physically to a rigid support to prevent it from collapsing. Also, the support must have a highly porous structure in order to be permeable to proteins. In addition, the mechanical fragility of the membranes hinders the industrial scale application of MEs.

Cretich et al., Electrophoresis, Wiley Interscience 24(4): 577-581 (2003), proposed substituting the continuous membranes of the prior-art with a bed of gel beads of identical comonomer composition, obtained by an inverse emulsion polymerization process. While the disclosed isoelectric beads conferred a stable pH without the mechanical fragility of membranes, the technique relies on an electric field to separate proteins in a mixture.

Accordingly, a need exists for more effective methods for separating proteins on the basis of pI value.

### SUMMARY

In one aspect, therefore, there is provided a chromatographic material comprising a solid buffer, said solid buffer comprising an amphoteric macromolecule that confers a predetermined pH to an aqueous solution, and (B) an ion exchange resin. and an ion exchange resin. In some embodiments, the solid buffer comprises a cross-linked polymer obtained from monomers of different pK. In others, the solid buffer and the ion exchange resin can be attached to the same or different solid supports. In one example, the solid supports are particles. In another, the solid support of the solid buffer comprises a substantially porous particle having a plurality of cavities extending inwardly from the surface. Solid supports also can comprise membranes or monoliths. In other embodiments, the solid buffer and ion exchange resin are not attached to a solid support.

In one example, a chromatographic material comprises a solid buffer (i) having an exclusion limit of lower than 5,000 Da and (ii) that is attached to a particle of diameter greater than about 50 µm. In another, a chromatographic material comprises a solid buffer (i) having an exclusion limit of 3,000 Da and (ii) that is attached to a particle of about 150 µm.

The ion exchange resins can comprise anion or cation exchangers.

In another aspect, an apparatus is provided that comprises a series of containers, wherein a first container in the series comprises a fluid inlet and a last container in the series comprises a fluid outlet, and each container in the series is in fluid communication with a next container in the series, and wherein each container in the series comprises a different chromatographic material comprising a solid buffer, said solid buffer comprising an amphoteric macromolecule that confers a predetermined pH to an aqueous solution, and (B) an ion exchange resin. and an ion exchange resin, and the containers are arranged in increasing or decreasing order according to the pH of the chromatographic material according to the type of ion exchange resin. When the solid buffer sequence from the top to the bottom is ordered under a pH decrease, the ion exchange resin is a cation exchanger. When the solid buffer sequence from the top to the bottom is ordered under a pH increase, the ion exchange resin is an anion exchanger. In one example, the solid buffer with the highest pH has a pH no greater than about pH 11, and the solid buffer with lowest pH has a pH no less than about pH 3.

In another example, the container is a well of a multi-well flow plate and each container in the series is connected by removable conduits. The containers also can comprise stackable cartridges which, when stacked, form a flow column.

In one example, the ion exchange resin is an anion exchanger and the containers are arranged in increasing order according to the pH of the solid buffer, while in another the ion exchange resin is a cation exchanger and the containers are arranged in decreasing order according to the pH of the solid buffer.

In another embodiment, proteins can be separated based on isoelectric point by (A) applying a mixture of two or more proteins to a series of chromatographic material, wherein each chromatographic material comprises (1) a solid buffer, said solid buffer comprising an amphoteric macromolecule that confers a predetermined pH to an aqueous solution, and (2) an ion exchange resin, and wherein the solid buffers are arranged in increasing or decreasing order according to the type of ion exchanger; (B) collecting flow-through from the last chromatographic material in the series; and (C) separately desorbing proteins from each chromatographic material.

Other objects, features and advantages of the present invention will become apparent from the following detailed description. Further, the examples demonstrate the principle of the invention.

### BRIEF DESCRIPTION OF THE DRAWINGS

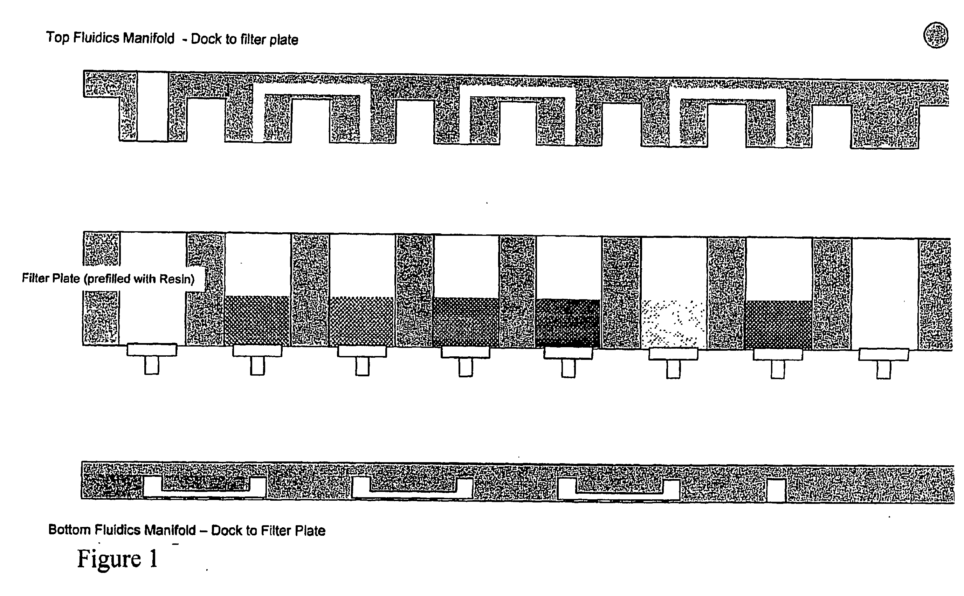

Figure 1 provides a schematic of a multi-well apparatus for separating proteins.

### DETAILED DESCRIPTION

This invention provides an ion exchange chromatographic material that operates at a pH selected by the operator. The chromatographic material comprises the combination of an ion exchange resin with a solid buffer, that stabilizes the pH of the solution in which it is placed. The ion exchange material is charged within the pH range generally used for chromatography, for example pH 3 to pH 11.

Proteins can be separated from mixtures based on their pI using a series of the chromatographic materials of this invention, each comprising a solid buffer and an ion exchange resin. Each solid buffer comprises an amphoteric macromolecule that confers a predetermined pH to an aqueous solution. Thus, each chromatographic material produces a particular pH. Proteins passing through, and possessing a pI different from that of, the chromatographic material will have either a net positive charge or a net negative charge or be neutral, depending on whether its pI is, respectively, below, above or the same as the pH of the chromatographic material. Proteins whose charge is opposite that of the ion exchange resin at the pH of the environment bind to the ion exchanger, while neutral or same charge proteins remain unbound and pass through the chromatographic material. That is, for example, a protein that is negatively charged at the pH of the solid buffer will bind to an anion exchange resin. Then, captured proteins can be eluted from the chromatographic material. In one embodiment of the invention, a series of chromatographic materials (mixture of solid buffer and ion exchange resin), each solid buffer producing a different pH, are arranged in series. Because different proteins are charged at different pH levels, ion exchange resins of each chromatographic material in the series captures a subset of the proteins in a mixture. In this fashion, proteins from biological fluids such as serum, urine, CSF, as well as soluble tissue extracts, can be separated as a function of their isoelectric point.

### 1. CHROMATOGRAPHIC MATERIALS

The chromatographic materials of this invention comprise a solid buffer in combination with an ion exchange resin. In preferred embodiments, each of these is attached to a solid phase. In particular, this invention contemplates a composition comprising solid buffer beads or particles mixed with ion exchange resin. In another embodiment, solid buffers and ion exchange resins are not attached to a solid phase. In another embodiment, buffering and ion exchange properties are associated within the same particle.

### 1.1 Solid buffers

The term "solid buffer" denotes an amphoteric, cross-linked, insoluble macromolecule that is obtained from ionisable monomers, each of which having a different pK. A solid buffer confers a predetermined pH to an aqueous solution of diluted electrolytes, and it maintains the pH when acidic or alkaline molecules are added. In addition to these physicochemical properties, a solid buffer useful in this document comprises small pores, giving the material a low exclusion limit, for example lower than 5000 Da.

A low exclusion limit prevents proteins from diffusing inside the cross-linked monomers while maintaining full diffusion for small ions. Such restricted diffusion minimizes the risk of non-specific binding to the solid buffer. In some embodiments, a solid buffer comprises an exclusion limit lower than 5000 Da, 4000 Da, or 3000 Da. Amphoteric macromolecules with small pores can be generated by using relatively concentrated monomer solutions and or high degrees of crosslinking monomers.

In one aspect, a solid buffer can be a polymer. For example, a solid buffer can be a polyacrylamide or a block copolymer. In another example, solid buffers can be made by combining acrylamide monomers of different pK to reach buffering power around at a predetermined pH.

Solid buffers can be prepared using routine chemicals and methods used in the arts of polymer chemistry and biochemistry. In general, to create a solid buffer for a particular pH, a monomer with a corresponding pK is selected, at a concentration that can range from few mM to several hundred mM, and is titrated to a pH, close or same to its pK with a complementary monomer (e.g. if the selected monomer has a pK of 8.0 it will be titrated to pH close to 8.0 using a monomer of a pK lower than 4.5). If a monomer of the desired pK is not available, a mixture of monomers (pK above and below the desired pK) can be used, followed by titration to a pH between the two pKs. Appropriate cross-linking reagents and polymerization catalysts are then added to the pH-adjusted solution of monomers in proportions sufficient to cause polymerization to generate particles of the solid buffer.

A variety of monomers are commercially available. Exemplarily monomers include, but are not limited to, N-acryloylglycine, 4-acrylamidobutyrric acid, 2-morpholinoethylacrylamide, 3-morpholinopropylacrylamide, N,N-dimethylaminoethylacrylamide and N,N-dimethylaminopropylacrylamide.

Immobilines also can be used to make solid buffers. Immobilines are acrylamide derivatives that conform to the general formula: 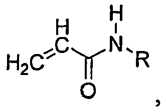 where R includes a group that provides the characteristic pI. *See*, *e.g.*, U.S. patent No. 4,971,670. While this characterization in principle embraces many molecules, Amersham produces molecules, marketed under the trademark IMMOBILINE®, that are particularly suited for creating isoelectric gels and polymers. The IMMOBILINE® collection of molecules includes the following, having the pI indicated in parenthetical: N-acryloylglycine (pK 3.6); 4-acrylamidobutyrric acid (pK 4.6); 2-morpholinoethyl-acrylamide (pK 6.2); 3-morpholinopropylacrylamide (pK 7.0); N,N-dimethylamino-ethylacrylamide (pK 8.5); and N,N-dimethylaminopropylacrylamide (pK 9.3) (collectively, "the immobilines"). Any of the immobilines can be combined, as monomers, and co-polymerized with acrylamide and N,N'-methylenebisacrylamide or another suitable cross-linking agent, to produce a desired pI specific polymer. Acrylamide can be substituted by other non-ionic acrylamide derivatives, such as N-isopropylacrylamide, methylacrylamide, methylolacrylamide dimethylacrylamide, diethylacrylamide, tris(hydroxymethyl)methylacrylamide, etc.

A variety of resources are available to assist in the selection of combinations and concentrations of monomers to produce a solid buffer with a particular pH. For example, formula tables for monomer combinations are provided in JOURNAL OF CHROMATOGRAPHIC LIBRARY, VOLUME 63 Chapter 12 (Righetti, Stoyanov & Zhukov eds., 2001). AMERSHAM BIOSCIENCES provides similar formula tables in its "Protocol Guide # 1: Isoelectric Membrane Formulas for IsoPrime Purification of Proteins." See:
htttp://www4.amershambiosciences.com/aptrix/upp00919.nsf/(FileDownload)?OpenA gent&docid=FD3302088BD37BC6C 1256EB400417E5C&file=80635018.pdf

Algorithms for selecting concentrations of monomers are available, too. *See*, *e.g.*, Giaffreda et al., J. Chromatog., 630:313-327 (1993). In addition, techniques for determining the pI for polymers are well-known in the art. Examples of such methods include Ribeiro et al., Computers in Biology & Medicine 20: 235-42 (1990), Ribeiro et al., loc. cit., 21: 131-41 (1991), and Sillero et al., Analytical Biochemistry 179: 319-25 (1989).

In one embodiment, the particles described in International Application PCT/US2005/007762 can be used as solid buffers.

In another embodiment, a solid buffer is polymerized within cavities of a solid support. In other embodiments, the solid buffer is deposited on the interior and exterior surfaces of a solid support, such as the interior and exterior surfaces formed by the interior pore volume of cavities in a particle. The deposition can be by chemical bond or other means.

Amino acids and peptides can provide such surface layers since they have defined isoelectric points. Thus, in some embodiments, a solid buffer comprises two or more amino acids. The amino acids can be any of the twenty naturally-occurring amino acids, or the amino acids can be synthetic amino acids. Useful amino acids include those along the twenty naturally occurring amino acids having ionizable side chains, including: lysine, arginine, glutamic acid, aspartic acid, serine, cysteine, threonine, tyrosine, asparagines, glutamine. In addition, it will be understood by those in the art that other compounds having defined pI values that can be attached to the interior and exterior particle surfaces as described above can be used with the present invention. Linkers can be used to provide attachment sites on the surface of a particle.

In general, a solid buffer should not be able to adsorb proteins by itself, should have the same density as the ion exchanger, and should have a good buffering capacity at the desired pH.

### 1.2 Solid support

The term "solid support" denotes a solid, porous material wherein ion exchange polymers or solid buffers can be attached or loaded to prevent polymer collapse under low concentration.

Chromatographic material can utilize a variety of solid supports. Illustrative of solid supports in this context are particles, membranes, and monoliths. A "monolith" is a single piece of material, generally porous, to which chromatographic ligands can be attached. Generally monoliths have significantly greater volume than beads, for example, in excess of 0.5 mL per cm³ of monolith.

In one embodiment, the solid support comprises a substantially porous particle having a plurality of cavities extending inwardly from the surface. The particles preferably have sizes, mechanical strengths and buoyancies that are compatible with separating biological extracts. In one embodiment, the particles comprise one or more mineral oxides such as silica, titania, zirconia, hafnia, alumina, gallia, , scandia, yttria, , actinia, or a rare earth mineral oxides.

When porous particles are employed as solid supports for solid buffers, relatively large particles can be used to minimize the external surface area relative to the particle volume and hence reduce the risk of non-specific binding for proteins. In some embodiments, the particles have diameters greater than about 50 µm, or greater than about 100 µm or greater than about 200 µm. In one example, particles of about 150 *µ*m are used as solid supports for solid buffers. Pore volume of the particle can range from 10 to 70% of the overall particle volume.

Alternatively, a porous, plastic bead can serve as solid support. Polystyrene is a well-known polymer that can be formed into beads having pores, for chromatography. Other synthetic polymers also can be used; for instance, those based on acrylics, such as methymethacrylates, as well as porous nylons, porous polyvinyl plastics, and polycarbonates. Additional examples of porous particle bodies are described in U.S. patents No. 6,613,234, No. 5,470,463, No. 5,393,430 and No. 5,445,732.

### 2. ION EXCHANGE RESINS

Ion exchange chromatography separates compounds based on their net charges. Negatively or positively charged functional groups are covalently bound to a solid support matrix, yielding either a cation or anion exchanger, respectively. When a charged compound is applied to an exchanger of opposite charge, it is adsorbed, while compounds that are neutral or the same have charge are eluted in the void volume of the column. Binding of the charged compounds is reversible, and adsorbed compounds are commonly eluted with a salt or a pH gradient.

The term "ion exchange resin" refers to a solid, porous network (mineral or organic or composite) carrying ionizable groups of positive or negative sign and of a single group. Positively charged ionic groups (anion exchangers) are, for example, quaternary, tertiary and secondary amines and pyridine derivatives. Negatively charged ionic groups (cation exchangers) are, for example, sulfonates, carboxylates and phosphates.

Selection of an ion exchange resins depends on the properties of the compounds to be separated. For amphoteric compounds, the pI of the compound and its stability at various pH values determine the separation strategy. At a pH above its pI, the compound of interest will be negatively charged, and at a pH below its pI the compound will be positively charged. Thus, if the compound is stable at a pH above its pI, an anion exchange resin is used. Conversely, if the compound is stable at a pH below its pI, a cation exchange resin is used. The operating pH also determines the type of exchanger to use. A strong ion exchange resin maintains capacity over a wide pH range, while a weak one loses capacity when the pH no longer matches the pKₐ of its functional group.

Anion exchangers can be classified as either weak or strong. The charge group on a weak anion exchanger is a weak base, which becomes deprotonated and, therefore, loses its charge at high pH. DEAE-cellulose is an example of a weak anion exchanger, where the amino group can be positively charged below pH ~ 9 and gradually loses its charge at higher pH values. A strong anion exchanger, on the other hand, contains a strong base such as a quaternary amine, which remains positively charged throughout the pH range normally used for ion exchange chromatography (pH 2-12).

Cation exchangers also can be classified as either weak or strong. A strong cation exchanger contains a strong acid (such as a sulfopropyl group) that remains charged from pH 1 - 14; whereas a weak cation exchanger contains a weak acid (such as a carboxymethyl group), which gradually loses its charge as the pH decreases below 4 or 4.5.

In one embodiment of the invention, strong ion exchangers such as quaternary amines or sulfonic acids are used. Weak ion exchangers, such as tertiary amines and carboxylic acids, also can be used, for example, when separating proteins that have pIs between 5 and 8.

**Table 1 provides a list of common ion exchangers.**

| Table 1. | |
|---|---|
| Strong anion | |
| -CH₂N⁺(CH₃)₃ | - Triethylaminomethyl |
| -C₂H₄N⁺(C₂H₅)₃ | - Triethylaminoethyl |
| -C₂H₄N⁺(C₂H5)₂CH₂CH(OH)CH₃ - | Diethyl-2-hydroxypropylaminoethyl |
| Weak anion | |
| -C₂H₄N⁺H₃ | - Aminoethyl |
| -C₂H₄NH(C₂H₅)₂ | - Diethylaminoethyl |
| Strong cation | |
| -SO³⁻ | - Sulpho |
| CH₂SO³⁻ | - Sulphomethyl |
| C₃H₆SO³⁻ | - Sulphopropyl |
| Weak cation | |
| -COO⁻ | - Carboxy |
| -CH₂COO⁻ | - Carboxymethyl |

Ion exchange resins are well known in the art. Commercially available ion exchangers useful in this invention include, but are not limited to, Q HyperD, S HyperD, Q Sepharose, S Sepharose, Q HyperZ and CM HyperZ. These resins can be mixed with solid buffer beads, for example, to produce the chromatographic materials of this invention.

Chromatographic material can comprise a volume of ion exchanger from 5% to 95% with the remainder being solid buffer.

### 3. USE OF CHROMATOGRAPHIC MATERIALS

Chromatographic material useful for separating proteins from mixtures based on their pI can be produced by combining a solid buffer with an ion exchange resin. In one embodiment, a solid buffer and ion exchange resin, each attached to different solid supports, are combined to form a mixture. In one aspect, the mixture is a bed of mixed particles.

In another embodiment, a chromatographic material comprises a solid buffer and an ion exchange resin attached to a single solid support, such as a membrane or monolith. In another example, an ion exchange resin is combined with a solid buffer on a single particle. In this embodiment, a solid buffer attached to a particle is prepared first, then a polymer with ion exchange properties and large pores is formed on top of the solid buffer.

Proteins passing through a chromatographic material of the invention will have either a net positive charge or a net negative charge or be neutral, depending on whether their pIs are, respectively, below, above or the same as the pH generated by the solid buffer. Proteins whose charge is opposite that of the ion exchange resin at the pH of the environment bind to the ion exchanger, while neutral or same charge proteins remain unbound and pass through the chromatographic material. That is, for example, a protein that is negatively charged at the pH of the solid buffer will bind to the ion exchange resin if this latter is an anion exchanger. Then, captured proteins can be eluted from the chromatographic material. Thus, the chromatographic material of the invention are useful for separating proteins based on pI.

In another embodiment, proteins are separated from mixtures based on their respective pI using a series of chromatographic material. In a multi-staged column comprised of different chromatographic material, a discontinuous gradient of pH is generated. Proteins in a mixture passing through such a column will become ionized differently according to their location in the column. When a protein obtains a charge opposite that of the ion exchange resin, it will bind to it. Thus, a mixture traveling through the column will be depleted of one protein category at a time as it crosses the different sections of the column.

Thus, a device of the invention can comprise a series of chromatographic materials comprising solid buffers of pH 9, 7 and 5, respectively (from the top to the bottom) and a cation exchanger. A sample of a biological extract is applied first to a container holding the solid buffer of 9. Proteins that have a pI above pH 9 will be positively charged in this environment and, therefore, will bind to the ion exchanger part of the chromatographic material. In most biological extracts this represents a minority of the protein species. The neutral or negatively charged proteins will not be bound and are eluted from the container. This eluate is then loaded to a container holding the solid buffer of pH 7. At this pH, the proteins having a pI above 7 will be positively charged and will bind to the ion exchanger. The neutral and positively charged proteins are unbound and are eluted from the container holding the chromatographic material. Accordingly, this section of chromatographic material has captured proteins having a pI between 7 and 9, with the proteins that have a pI above 9 having been captured already, on the first section of chromatographic material. Then, this second eluate is loaded to a third container holding chromatographic material with solid buffer of pH 5. According to a similar mechanism described above, proteins of pI between 5 and 7 will be captured by the cation exchanger, Proteins with pI at or below 5 will not be captured and will be found in the eluate. The bound proteins can be eluted thereafter, by conventional means, from the various containers. Accordingly, the proteins have been fractionated according to pI, into fractions having pI above 9, pI between 7 and 9, pI between 5 and 7 and pI below 5. In a similar way, proteins can be fractionated by cation exchange chromatography, using chromatographic materials composed of solid buffers of increasing pH.

In one aspect, the series can comprise two, three, four, five, six, seven, eight, nine, ten, eleven or twelve different chromatographic materials. As described above, the chromatographic materials are aligned in order of increasing or decreasing pH depending on whether an anion exchanger or a cation exchanger, respectively, is used.

In preparing a solution for loading onto a chromatographic adorbent, electrolytes such as simple salts, for example, sodium chloride or potassium chloride, can be used. As biological molecules can act as electrolytes, however, neat water can be used. Modifiers also can be added to a mixture to prevent proteins from aggregating. Examples of such modifiers include, but are not limited to, glycols and non ionic chaotropic agents, such as urea or non-ionic detergents.

Bound proteins can be desorbed using any chemical component capable of eluting proteins from an ion exchange resin. Most generally, salt solutions are used to desorb proteins; however, a pH change also can be used, as well as displacers.

In another embodiment, there is provided an apparatus comprising a series of containers, wherein a first container in the series comprises a fluid inlet and a last container in the series comprises a fluid outlet, and each container in the series is in fluid communication with a next container in the series, and wherein each container in the series comprises a different chromatographic material and the containers are arranged in increasing or decreasing order according to the pH of the chromatographic materials.

In one embodiment, the chromatographic materials are contained within cartridge or container segments having inlets and outlets. The segments are stackable with and detachable from each other. The segments each contain a different chromatographic material. They can contain filters or membranes that hold the Chromatographic material in place. When attached end-to-end, the segments create a column into which a solution can be poured. After the fluid has passed through all of the segments, the segments can be detached from one another and the captured proteins eluted from each segment. See, for example, WO 03/036304 (Schultz et al.)

In another example, the container is a well of a multi-well flow plate and wherein each container in the series is connected by removable conduits. (Figure 1). In one aspect, the plate can be a microtiter plate such as drip plate or a filter plate. In other embodiments, however, the plate can comprise a piece comprising channels, such as bores, that open on either side of the piece and that will define chambers when conduits are attached to the openings of the bores. Preferably, the chambers are arrayed substantially in a plane. Exemplary devices are described in WO2006/127056 which claims priority of U.S. provisional application No. 60/684177.

In the multi-well flow plate, the combination of chambers and conduits define a fluid path whereby a fluid can be pumped from chamber to chamber. Each chamber in the series comprises a different chromatographic material that can capture a different subset of analytes in a complex sample. A particular utility of this device is that the conduits are removable so that analytes captured by a chromatographic material can be isolated by, e.g., eluting the analytes from the chambers. Once isolated, the analytes can be detected or analyzed by any available methodology.

In one example of such an embodiment, the fluid is forced alternately down through one chamber in the series (e.g., a column or a row), up through the next chamber in series, down through the next chamber in the series, up through the next chamber in the series, and so on. More specifically, the bottom opening of a first chamber in a series (e.g., column or row) may be connected to the bottom opening of a second chamber in that series. The top opening of the second chamber may be connected to the top opening of a third chamber in that row (or column). The sequencing of connecting bottom-to-bottom and top-to-top generates a flow path that travels from top-to-bottom of the first chamber, then bottom-to-top of second chamber, and then top-to-bottom of third chamber, etc.

In another example, the fluid is forced down through each chamber in the series. More specifically, the bottom opening of a first chamber in a column (or row) may be connected to the top opening of a second chamber in that column (or row). The bottom opening of the second chamber may be connected to the top opening of a third chamber in that row (or column). The sequencing of connecting bottom-to-top generates a flow path that travels from top-to-bottom of the first chamber, top-to-bottom of second chamber, top-to-bottom of third chamber, etc.

In addition, the materials, methods and apparatuses described herein can be used in combination with additional materials and apparatuses to provide additional analytical information about a separated protein. For example, separated proteins can be subjected to either further separations or analysis by gel electrophoresis or mass spectrometry, e.g., SELDI. In this fashion, a separated protein can effectively be identified.

It is understood that the examples and embodiments described herein are for illustrative purposes only.

### Example 1. Preparation of solid buffer of pH 8.5

A solid-phase buffer of pH 8.5 can be prepared by dissolving 10 mMoles of N,N-dimethyl-aminopropyl-acrylamide having a pK of 8.5 (this is 1420 mg of free base) in 50 mL of water. The solution is titrated to pH 8.5 by slow addition of the monomer acrylamidoglycolic acid of pK 3.1 (free acid). Next, 30 g of acrylamide and 2 g of methylene-bis-acrylamide are added to the solution. The volume of the solution is then raised to 100 ml. To this final solution, polymerization catalysts are added, for example ammonium persulfate and TEMED. The solution is then used, for example, to impregnate porous particles. Once the hydrogel has polymerized inside the pores of the particles, the material is washed to remove by-products and reagent excess. The solid-phase buffer can be stored in the presence of 20% ethanol.

### Examples 2. Preparation of solid buffer of pH 4.6

A solid-phase buffer of pH 4.6 can be prepared by dissolving 100 mM of N-acryloyl glycine (pK 4.6) in 1 liter of distilled water. The solution is then titrated to pH 4.6 by adding a concentrated solution (50% in water) of N,N,N-triethyl aminoethyl acrylamide. To the obtained solution, 200 gram of acrylamide and 10 grams of N,N'-methylene-bis-acrylamide are added. The mixture is stirred to complete solubilization. A catalysis system composed of N,N,N',N'-tetramethylethylene diamine and ammonium persulfate is added to the solution just before use. 38 ml of the final solution is added to 100 ml of porous zirconia beads of about 75 µM (pore volume of 38 ml for 100 gram) and mixed to the complete absorption of the solution in the porous volume of the mineral beads. Next, the impregnated beads are degassed under vacuum three times, alternating the introduction of nitrogen. The mixture is then stored at room temperature in the presence of nitrogen until polymerization of the monomers. The resulting solid buffer is washed extensively with water, with a buffer of the same pH (e.g. acetate buffer pH 4.6) and finally with water. The solid buffer is then ready for use in the presence of an ion exchanger.

### Example 3. Preparation of solid buffer of pH 9.3

A solid buffer of pH 9.3 can be prepared by dissolving 100 mM of N,N-dimethyl aminopropyl acrylamide (pK 9.3) in 1 liter of distilled water. The solution is then titrated to pH 9.3 by adding a concentrated solution (50% in water) of acrylic acid. To the obtained solution, 200 gram of dimethyl-acrylamide and 10 grams of N,N'-methylene-bis-acrylamide are added. The resulting mixture is stirred to complete solubilization. A catalysis system composed of N,N,N',N'-tetramethylethylene diamine and ammonium persulfate is added just before use. 38 ml of the final solution is added to 100 ml of porous zirconia beads of about 75 µM (pore volume of 38 ml for 100 gram) and mixed to complete absorption of the solution in the porous volume of the beads. Next, the impregnated beads are degassed under vacuum three times, alternating the introduction of nitrogen. The mixture is then stored at room temperature in the presence of nitrogen until polymerization of monomers. The resulting material is washed extensively with water, a buffer of the same pH (*e.g.* Tris-HCl buffer, pH 9.3) and water again. The solid buffer is then ready for use in the presence of an ion exchanger.

### Example 4. Preparation of solid buffer of pH 7.7

A solid buffer of pH 9.3 can be prepared by dissolving 50 mM of 3-morpholinopropyl acrylamide (pK 7.0) and 50 mM of N,N-dimethylaminoethyl acrylamide (pK 8.5) in 1 liter of distilled water. The solution is then titrated to pH 7.7 by adding a concentrated solution (50% in water) of 2-acrylamido-2-methylpropane sulfonic acid. To the obtained solution, 200 gram of acrylamide and 10 grams of N,N'-methylene-bis-acrylamide are added, and the resulting mixture is stirred to complete solubilization. A catalysis system composed of N,N,N',N'-tetramethylethylene diamine and ammonium persulfate is added just before use. 38 ml of the final solution is added to 100 ml of porous zirconia beads of about 75 µM (pore volume of 38 ml for 100 gram) and mixed to the complete absorption of the solution in the porous volume of the beads. Next, the impregnated beads are degassed under vacuum three times, alternating the introduction of nitrogen. The mixture is then stored at room temperature in the presence of nitrogen until polymerization of monomers. The resulting material is washed extensively with water, a buffer of the same pH (e.g. morpholine-HCl buffer pH 7.7) and water again. The solid buffer is then ready for use in the presence of an tion exchanger.

### Example 5. Preparation of a mix mode chromatographic material (solid buffer of pH 4.6 and cation exchanger)

A solid buffer of pH 4.6 can be prepared by dissolving 150 mM of N-acryloyl glycine (pK 4.6) and 10 mM N,N'-methylene-bis-acrylamide in 1 liter of distilled water. The solution is then titrated to pH 4.6 by adding a concentrated solution (50% in water) of N,N,N-triethyl aminoethyl acrylamide. A catalysis system composed of N,N,N',N'-tetramethylethylene diamine and ammonium persulfate is added just before use.

Separately a second aqueous solution composed of 5% of 2-acrylamido-2-methylpropane sulfonic acid sodium salt, 5% of dimethylacrylamide and 1% of and mM N,N'-methylene-bis-acrylamide is prepared. The pH of this solution is then adjusted to 4.6 by addition of a base or an acid. A catalysis system composed of N,N,N',N'-tetramethylethylene diamine and ammonium persulfate is added just before use.

100 ml of porous zirconia beads of about 75 µM (pore volume of 40 ml for 100 gram) are mixed with 20 ml of the first monomer solution and then treated as described in the previous examples up to the polymerization. This intermediate product is washed extensively with water, with a buffer of the same pH (e.g. acetate buffer pH 4.6) and finally with water. The washed product is then dried using for example repeated washes with dry ethanol and acetone.

The intermediate dry product is then mixed with 20 ml of the second monomer solution (to fill up the total porous volume of the mineral beads). A second polymerization process is then started as above. The final chromatographic material is washed extensively with water, with acetate buffer pH 4.6 and distilled water.

### Example 6. Preparation of solid buffer of pH 6.5

A solid buffer of pH 6.5 can be prepared by dissolving 150 mM of 3-morpholinopropyl acrylamide (pK 7.0) in 1 liter of distilled water. The solution is then titrated to pH 6.5 by adding a concentrated solution (50% in water) of N-acryloyl glycine (pK 3.6). To the obtained solution 400 gram of acrylamide and 30 grams of N,N'-methylene-bis-acrylamide are added, and the resulting mixture is stirred to complete solubilization. A catalysis system composed ofN,N,N',N'-tetramethylethylene diamine and ammonium persulfate is added just before use. 100 ml of this solution is dispersed in 500 ml of paraffine oil containing 3% of arlacel C (oil-soluble emulsifier). The suspension is maintained under stirring for 3 hours at 65°C to allow monomers to copolymerize together. Hydrogel beads formed during polymerization are collected by filtration and washed extensively with a non-polar solvent to eliminate traces of paraffin oil. Next, the beads are washed extensively with water, with a buffer of the same pH and finally with water. The solid buffer is then ready for use in the presence of an ion exchanger.

### Example 7. Preparation of solid buffer of pH 9.0 using irregular particles

A solid buffer of pH 6.5 can be prepared by dissolving 150 mM of N,N,N-triethyl-aminoethyl-acrylamide (pK 12) in 1 liter of distilled water. The solution is then titrated to pH 9.0 by adding a concentrated solution (50% in water) of acrylamidoglycolic acid (pK 3.1). To the obtained solution, 300 gram of dimethyl-acrylamide and 20 grams of N,N'-methylene-bis-acrylamide are added, and the resulting mixture is stirred to complete solubilization. A catalysis system composed of N,N,N',N'-tetramethylethylene diamine and ammonium persulfate is added just before use. The solution then is placed in a warm bath of 65°C under nitrogen. Five hours later the polymerization is complete under a full hydrogel block. The hydrogel is cut into small pieces and ground to get particles of about 100 µm. Particulated product is then washed extensively with water, then with a buffer of the same pH and finally with water. The solid buffer is then ready for use in the presence of a ion exchanger.

### Example 8. Separation of proteins based on pI using an anion exchanger mixed with three different solid buffers.

A series of three chromatographic materials are assembled in a series of interconnected sectional columns (outlet of the previous to the inlet of the following) column. Each of the chromatographic materials comprises the same anion exchanger (Q HyperZ sorbent) but a different solid buffer of different pH, in particular, 5.4, 7.7 and 9.5, respectively. The chromatographic sectional columns are aligned in order of increasing pH, *i.e.* the first sectional column has a pH of 5.4 and the last adsorbent has pH of 9.5.

The Q anionic sorbent is positively charged in all pH ranges induced by the solid-phase buffers (5.4 to 9.5).

A sample containing five proteins (lysozyme (pI 11), cytochrome c (pI 9.0), myoglobin (pI 7.0), human albumin (pI 6.0) and fetuin (pI ≤ 5.0)) is prepared in 10 mM potassium chloride. To avoid protein-protein interaction, 2M urea is added. The sample is then loaded onto the column.

At the first chromatographic material, only fetuin adsorbs because it is the only protein negatively charged at pH 5.4. The remaining four proteins will progress to the second chromatographic material.

At the second chromatographic material, both albumin and myoglobin are negatively charged at pH 5.4. Thus, they are captured by the anion exchanger.

At the third chromatographic material, only cytochrome C adsorbs because it is the only protein remaining in the sample that is negatively charged at pH 9.5.

Lysozyme possesses a net positive charge at pH 9.5. Accordingly, it will exit the column with the flow-through.

Once the adsorption phase is over, the chromatographic materials are disconnected and separately treated to desorb proteins. The bound proteins are desorbed using 1 M potassium chloride.

### Example 9. Separation of proteins based on pI using a cation exchanger mixed with three different solid buffers

A series of three chromatographic materials are assembled in a series of interconnected sectional columns (outlet of the previous to the inlet of the following) column. Each of the chromatographic materials comprises the same cation exchanger but a different solid buffer of different pH, in particular, 5.4, 7.7 and 9.5, respectively. The chromatographic sectional columns are aligned in order of decreasing pH, *i.e.* the first sectional column has a pH of 9.5 and the last adsorbent has a pH of 5.4.

The cation exchanger is negatively charged in all pH ranges induced by the solid-phase buffers (5.4 to 9.5).

A sample containing five proteins (lysozyme (pI 11), cytochrome c (pI 9.0), myoglobin (pI 7.0), human albumin (pI 6.0) and fetuin (pI ≤ 5.0)) is prepared in 10 mM potassium chloride. To prevent protein-protein interaction, 2M urea is added. The sample is then loaded onto the column.

At the first chromatographic material, only lysozyme adsorbs because it is the only protein negatively charged at pH 9.5. The remaining four proteins progress to the second chromatographic material.

At the second chromatographic material, only cytochrome C adsorbs because it is the only protein remaining in the sample that is negatively charged at pH 7.7. The remaining proteins progress to the third chromatographic material.

At the third chromatographic material, both albumin and myoglobin are negatively charged at pH 5.4. Thus, these proteins are captured by the anion exchanger.

Fetuin possesses a net positive charge at pH 5.4. Thus, fetuin will exit the column with the flow-through.

Once the adsorption phase is over, the chromatographic materials are disconnected and separately treated to desorb proteins. The bound proteins are desorbed using 1 M potassium chloride.

## Claims

1. A chromatographic material comprising (A) a solid buffer, said solid buffer comprising an amphoteric macromolecule that confers a predetermined pH to an aqueous solution, and (B) an ion exchange resin.

2. The chromatographic material of claim 1, wherein the solid buffer and the ion exchange resin are attached to different solid supports.

3. The chromatographic material of claim 1, wherein
(i) the solid buffer and the ion exchange resin are not attached to solid supports; or
(ii) the solid buffer and the ion exchange resin are attached to a single solid support.

4. The chromatographic material of claim 2, wherein
(i) the solid buffer has an exclusion limit of lower than 5,000 Da and is attached to a particle of greater than about 50 µm; or
(ii) the solid buffer has an exclusion limit of 3,000 Da and is attached to a particle of about 150 µm; or
(iii) the solid supports are particles, membranes or monoliths, preferably the solid supports are particles.

5. The chromatographic material of claim 1, wherein
(i) the solid buffer comprises a crosslinked polymer obtained from monomers of different pK; or
(ii) the solid support of the solid-phase buffer comprises a substantially porous particle having a plurality of cavities extending inwardly from the surface; or
(iii) the ion exchange resin is an anion exchanger; or
(iv) the ion exchange resin is a cation exchanger.

6. An apparatus comprising a series of containers, wherein a first container in the series comprises a fluid inlet and a last container in the series comprises a fluid outlet, and each container in the series is in fluid communication with a next container in the series, and wherein each container in the series comprises a different chromatographic material according to claim 1 and the containers are arranged in increasing or decreasing order according to the pH of the chromatographic material.

7. The apparatus of claim 6, wherein
(i) the container is a well of a multi-well flow plate and wherein each container in the series is connected by removable conduits; or
(ii) the containers comprise stackable cartridges which, when stacked, form a flow column.

8. The apparatus of claim 6, wherein the solid buffer with the highest pH has a pH no greater than about pH 11, and the solid buffer with lowest pH has a pH no less than about pH 3.

9. The apparatus of claim 6, wherein
(i) the ion exchange resin is an anion exchanger and the containers are arranged in increasing order according to the pH of the chromatographic material; or
(ii) the ion exchange resin is a cation exchanger and the containers are arranged in decreasing order according to the pH of the chromatographic material.

10. A method of separating proteins based on isoelectric point, comprising
(A) applying a mixture of two or more proteins to a series of chromatographic material, wherein each chromatographic material comprises a solid buffer and an ion exchange resin, said solid buffer comprising an amphoteric macromolecule that confers a predetermined pH to an aqueous solution, and wherein the chromatographic materials are arranged in increasing or decreasing order according to the pH of the chromatographic material,
(B) collecting flow-through from the last chromatographic material in the series, and then
(C) separately desorbing proteins from each chromatographic material.

## Patentansprüche

1. Chromatographisches Material, umfassend (A) einen festen Puffer, wobei der feste Puffer ein amphoteres Makromolekül umfasst, das einer wässrigen Lösung einen vorbestimmten pH-Wert verleiht, und (B) ein Ionenaustauscherharz.

2. Chromatographisches Material gemäß Anspruch 1, wobei der feste Puffer und das Ionenaustauscherharz an verschiedene feste Träger gebunden sind.

3. Chromatographisches Material gemäß Anspruch 1, wobei
(i) der feste Puffer und das Ionenaustauscherharz nicht an feste Träger gebunden sind; oder
(ii) der feste Puffer und das Ionenaustauscherharz an einen einzigen festen Träger gebunden sind.

4. Chromatographisches Material gemäß Anspruch 2, wobei
(i) der feste Puffer eine Ausschlussschwelle von weniger als 5000 Da aufweist und an ein Teilchen von mehr als etwa 50 µm gebunden ist; oder
(ii) der feste Puffer eine Ausschlussschwelle von 3000 Da aufweist und an ein Teilchen von etwa 150 µm gebunden ist; oder
(iii) die festen Träger Teilchen, Membranen oder Monolithe sind, wobei die festen Träger vorzugsweise Teilchen sind.

5. Chromatographisches Material gemäß Anspruch 1, wobei
(i) der feste Puffer ein vernetztes Polymer umfasst, das aus Monomeren mit unterschiedlichen pK-Werten erhalten wurde; oder
(ii) der feste Träger des Festphasenpuffers ein im Wesentlichen poröses Teilchen umfasst, das eine Vielzahl von Hohlräumen aufweist, die sich von der Oberfläche aus nach innen erstrecken; oder
(iii) das Ionenaustauscherharz ein Anionenaustauscher ist; oder
(iv) das Ionenaustauscherharz ein Kationenaustauscher ist.

6. Vorrichtung, die eine Reihe von Behältern umfasst, wobei ein erster Behälter in der Reihe einen Fluideinlass umfasst und ein letzter Behälter in der Reihe einen Fluidauslass umfasst und jeder Behälter in der Reihe in Fluidkommunikation mit einem nächsten Behälter in der Reihe steht und wobei jeder Behälter in der Reihe ein anderes chromatographisches Material gemäß Anspruch 1 umfasst und die Behälter in aufsteigender oder absteigender Reihenfolge gemäß dem pH-Wert des chromatographischen Materials angeordnet sind.

7. Vorrichtung gemäß Anspruch 6, wobei
(i) der Behälter ein Napf einer Multinapf-Durchflussplatte ist und wobei jeder Behälter in der Reihe durch entnehmbare Leitungen verbunden ist; oder
(ii) die Behälter stapelbare Kartuschen umfassen, die, wenn sie gestapelt sind, eine Durchflusssäule bilden.

8. Vorrichtung gemäß Anspruch 6, wobei der feste Puffer mit dem höchsten pH-Wert einen pH von nicht mehr als etwa pH 11 aufweist und der feste Puffer mit dem niedrigsten pH-Wert einen pH von nicht weniger als etwa pH 3 aufweist.

9. Vorrichtung gemäß Anspruch 6, wobei
(i) das Ionenaustauscherharz ein Anionenaustauscher ist und die Behälter in aufsteigender Reihenfolge gemäß dem pH-Wert des chromatographischen Materials angeordnet sind; oder
(ii) das Ionenaustauscherharz ein Kationenaustauscher ist und die Behälter in absteigender Reihenfolge gemäß dem pH-Wert des chromatographischen Materials angeordnet sind.

10. Verfahren zum Trennen von Proteinen auf der Basis des isoelektrischen Punkts, umfassend:
(A) Auftragen eines Gemischs von zwei oder mehr Proteinen auf eine Reihe von chromatographischen Materialien, wobei jedes chromatographische Material einen festen Puffer und ein Ionenaustauscherharz umfasst, wobei der feste Puffer ein amphoteres Makromolekül umfasst, das einer wässrigen Lösung einen vorbestimmten pH-Wert verleiht, und wobei die chromatographischen Materialien in aufsteigender oder absteigender Reihenfolge gemäß dem pHWert des chromatographischen Materials angeordnet sind;
(B) Auffangen der durchfließenden Flüssigkeit aus dem letzten chromatographischen Material in der Reihe und dann
(C) getrenntes Desorbieren von Proteinen aus jedem chromatographischen Material.

## Revendications

1. Matériau chromatographique comprenant (A) un tampon solide, ledit tampon solide comprenant une macromolécule amphotère qui confère un pH prédéterminé à une solution aqueuse, et (B) une résine échangeuse d'ions.

2. Matériau chromatographique selon la revendication 1, dans lequel le tampon solide et la résine échangeuse d'ions sont fixés sur différents supports solides.

3. Matériau chromatographique selon la revendication 1, dans lequel
(i) le tampon solide et la résine échangeuse d'ions ne sont pas fixés sur des supports solides ; ou
(ii) le tampon solide et la résine échangeuse d'ions sont fixés sur un support solide unique.

4. Matériau chromatographique selon la revendication 2, dans lequel
(i) le tampon solide a une limite d'exclusion inférieure à 5 000 Da et est fixé à une particule supérieure à environ 50 µm ; ou
(ii) le tampon solide a une limite d'exclusion de 3 000 Da et est fixé à une particule d'environ 150 µm ; ou
(iii) les supports solides sont des particules, des membranes ou des monolithes, et de préférence, les supports solides sont des particules.

5. Matériau chromatographique selon la revendication 1, dans lequel
(i) le tampon solide comprend un polymère réticulé obtenu à partir de monomères de pK différents ; ou
(ii) le support solide du tampon en phase solide comprend une particule essentiellement poreuse présentant plusieurs cavités qui s'étendent vers l'intérieur depuis la surface ; ou
(iii) la résine échangeuse d'ions est une résine échangeuse d'anions ; ou
(iv) la résine échangeuse d'ions est une résine échangeuse de cations.

6. Appareil comprenant une série de conteneurs, dans lequel un premier conteneur de la série comprend une admission de fluide et un dernier conteneur de la série comprend une sortie de fluide, et chaque conteneur de la série est en communication fluide avec le conteneur suivant de la série, et dans lequel chaque conteneur de la série comprend un matériau chromatographique différent selon la revendication 1 et les conteneurs sont disposés dans un ordre croissant ou décroissant en fonction du pH du matériau chromatographique.

7. Appareil selon la revendication 6, dans lequel
(i) le conteneur est un puits d'une plaque d'écoulement multi-puits dans laquelle chaque conteneur de la série est connecté par des conduits amovibles ; ou
(ii) les conteneurs comprennent des cartouches empilables qui, lorsqu'elles sont empilées, forment une colonne d'écoulement.

8. Appareil selon la revendication 6, dans lequel le tampon solide ayant le plus grand pH a un pH n'excédant pas environ pH 11, et le tampon solide ayant le plus faible pH a un pH qui n'est pas inférieur à environ pH 3.

9. Appareil selon la revendication 6, dans lequel
(i) la résine échangeuse d'ions est une résine échangeuse d'anions et les conteneurs sont disposés dans un ordre croissant en fonction du pH du matériau chromatographique ; ou
(ii) la résine échangeuse d'ions est une résine échangeuse de cations et les conteneurs sont disposés dans un ordre décroissant en fonction du pH du matériau chromatographique.

10. Procédé de séparation de protéines sur la base du point isoélectrique, comprenant les étapes consistant à
(A) appliquer un mélange de deux protéines ou plus à une série de matériau chromatographique, où chaque matériau chromatographique comprend un tampon solide et une résine échangeuse d'ions, ledit tampon solide comprenant une macromolécule amphotère qui confère un pH prédéterminé à une solution aqueuse, et où les matériaux chromatographiques sont disposés dans un ordre croissant ou décroissant en fonction du pH du matériau chromatographique,
(B) collecter l'écoulement depuis le dernier matériau chromatographique de la série, puis
(C) désorber séparément les protéines de chaque matériau chromatographique.
